# EUROPÄISCHE PATENTSCHRIFT

(11) **EP 1 985 747 B1**
(45) Veröffentlichungstag und Bekanntmachung des Hinweises auf die Patenterteilung: **26.10.2011**
(21) Anmeldenummer: 08007534.4
(22) Anmeldetag: 17.04.2008
(51) Int. Cl.: D06M 23/16, D06Q 1/10, D04H 3/14, D04H 1/54, A61F 13/15

(54) **Vliesstoff mit verbesserten Maskierungseigenschaften gegenüber Körperflüssigkeiten**
Non-woven fabric with improved masking characteristics for bodily fluids
Tissu non tissé doté de caractéristiques de masquage améliorées par rapport aux liquides corporels

(30) Priorität: 23.04.2007 DE 102007019427
(43) Veröffentlichungstag der Anmeldung: 29.10.2008
(73) Patentinhaber: Sandler AG, 95126 Schwarzenbach/Saale (DE)
(72) Erfinder:

(56) Entgegenhaltungen:
- WO-A1-97/00656
- WO-A1-98/56326
- US-A- 6 025 535

## Beschreibung

Die Erfindung betrifft Vliesstoffe zur Verwendung in absorbierenden Einwegartikeln, wie sie im Medizin- und/oder Hygienebereich eingesetzt werden, also zum Beispiel Damenbinden oder Slipeinlagen, Windeln, aber auch Pflaster, Wundabdeckungen und dergleichen.

In der folgenden Beschreibung und in den Beispielen wird sich der Einfachheit halber häufig auf die Anwendung des erfindungsgemäßen absorbierenden Artikels als Damenbinde bezogen. Dies ändert aber nichts an der Tatsache, dass auch die weiteren beispielsweise eingangs aufgeführten absorbierenden Artikel im Sinn dieser Erfindung ausgestaltet werden können.

Absorbierende Einwegartikel sind generell schichtenweise aufgebaut und bestehen meist aus
➢ einer körperseitigen, flüssigkeits- und luftdurchlässigen Abdeckschicht, dem sogenannten Topsheet.
➢ aus einer der Bekleidung zugewandten flüssigkeitsdichten Abdeckschicht, dem sogenannten Backsheet und
➢ einem zwischen diesen Schichten angeordneten absorbierenden Kern.

Absorbierende Einwegartikel funktionieren, vereinfacht dargestellt, in der Weise, dass die vom Körper abgegebenen Flüssigkeiten - beispielsweise Urin oder Blut - das körperseitig zugewandte, flüssigkeitsdurchlässige Topsheet durchdringen und im absorbierenden Kern aufgenommen und festgehalten werden. Das bekleidungsseitig zugewandte flüssigkeitsundurchlässige Backsheet verhindert, dass die vom Kern aufgenommenen Flüssigkeiten den absorbierenden Artikel verlassen und die Kleidung des Trägers verschmutzen können.

Aus Gründen der Optik und des Komforts, speziell bei Slipeinlagen und Binden, wünscht der Träger, dass Körperflüssigkeiten wie Urin oder Blut das Topsheet schnell durchdringen, im absorbierenden Kern gespeichert werden und möglichst nicht mehr auf dem Topsheet sichtbar, das heißt maskiert sind.

In der Vergangenheit gab es vielfältige Anstrengungen, was die Auswahl der hierfür eingesetzten Materialien bzw. deren Verbesserung anbelangt.

Ein Ansatz ist die Verwendung von gelochten Folien als Topsheet. Die zum Einsatz kommenden Folien bestehen zumeist aus Polyethylen und werden mit Mattierungsmitteln wie Titandioxid oder anderen Farbstoffen bei der Extrusion bereits eingefärbt. Um die an sich geschlossene Folienfläche flüssigkeitsdurchlässig zu gestalten, werden in die Folie Löcher eingebracht, durch die beim Gebrauch die Körperflüssigkeiten von der Oberfläche des Topsheet in den absorbierenden Kern gelangen. Gattungsbildend hierzu ist die US 6228462.

Obwohl mittels dieser Technik gute Maskierungseigenschaften zu erreichen sind, hat das Material aufgrund fehlender textiler Eigenschaften einen folienartigen Griff, wodurch die Trageeigenschaften negativ beeinflusst werden. Auch sind die Flüssigkeitsaufnahme und die Zeit, die dafür benötigt wird, nur durch größere Löcher bzw. vermehrte Lochanzahl steuerbar. Dies geht zu Lasten der mechanischen Stabilität und auch, da sich die offene Fläche erhöht, auch zu Lasten der Abdeckwirkung, was gelochte Folie für diesen Einsatzzweck disqualifiziert.

Eine weitere Möglichkeit zur Verbesserung der Maskierungseigenschaften von Hygieneprodukten ist es, eine Kombination von Vlies mit Folie einzusetzen. Als dem Träger zugewandtes Material wird zur Verbesserung der Griffeigenschaften ein Vliesstoff verwendet, dem in Richtung des Saugkerns eine Folie nachgeschaltet ist. Ein Aufbau dieser Art wird beispielsweise in US 3967623 beschrieben. Nachteilig ist hier neben der mehrlagigen Arbeitsweise, dass die dem Träger zugewandte Seite der Topsheet-Komponente vollständig hydrophiler Natur ist. Flüssigkeiten werden dadurch zwar schnell aufgenommen, verteilen sich aber auf der Oberfläche des Topsheets - die Optik im Gebrauch leidet darunter.

**Die** US 6025535 **beschreibt das Aufbringen von Tropfen eines die Oberflächenspannung modifizierenden Mittels auf einen Vliesstoff. Die Tropfen sind dabei wirr über den Vliesstoff verteilt und auch nur einseitig vorhanden. Dadurch wird lediglich ein Anschmutzen verhindert, eine effektive Maskierung wird dabei nicht erreicht.**

Die WO 97/00656**,** WO 98/56326**,** EP 633762 und auch die DE 4321155 beschreiben das definierte, d.h. das mustermäßige Aufdrucken von Substanzen, die nach dem Abkühlen oder Aushärten Fluid-undurchlässige Flächenbereiche auf einem Topsheet erzeugen. Nachteilig ist dabei, dass in den so bedruckten Bereichen, die das Ausgangsmaterial bildenden Fasern miteinander verklebt werden und die so bedruckten Bereiche tatsächlich folienartig dicht verschlossen werden. Obgleich maskierende Eigenschaften erzielt werden, verringert sich dadurch die Atmungsaktivität, ausgedrückt als Durchlässigkeit gegenüber gasförmigen Fluiden, und damit die Trageeigenschaften eines so hergestellten Hygieneprodukts wie einer Slipeinlage. Darüber hinaus wirkt sich diese Versiegelung ebenfalls negativ auf die Weichheit des Topsheets aus. Die Fähigkeit des Topsheets, Fluide von der dem Körper zugewandten Seite in den absorbierenden Kern passieren zu lassen, wird deutlich herabgesetzt, da ein Großteil der zur Verfügung stehenden Fläche, wie eingangs erwähnt quasi versiegelt wird.

Aufgabe der vorliegenden Erfindung ist es daher, einen verbesserten Vliesstoff für absorbierende Einwegartikel bereitzustellen, der die Nachteile des Stands der Technik nicht aufweist.

Gelöst wird die Aufgabe durch die Merkmale des Anspruchs 1, vorteilhafte Ausgestaltungen sind in den Unteransprüchen 2 bis 15 genannt.

Der erfindungsgemäße Vliesstoff (1) stellt eine bisher nicht erreichbare Balance der an sich konträr verlaufenden Eigenschaften
➢ Flüssigkeitstransport
➢ Maskierungseigenschaften
➢ Atmungsaktivität
➢ Hautverträglichkeit- freundlichkeit, Weichheit/ Tragekomfort
her. Bei der vorliegenden Erfindung wird diese Balance durch eine partielle Erhöhung der Opazität und damit einhergehend auch einer partiellen Erhöhung der Hydrophobie von an sich hydrophilen Vliesstoffen erzielt, ohne die Durchlässigkeit gegenüber Fluiden zu beeinflussen.

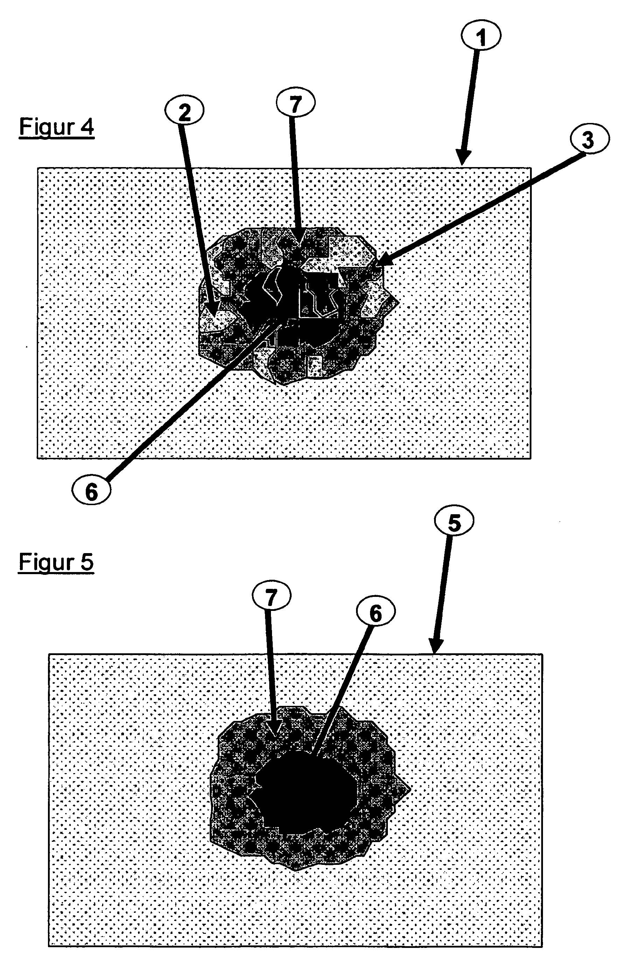

Als Opazität wird im Rahmen der Erfindung die Eigenschaft eines Vliesstoffes bezeichnet, die den Farbtiefeneindruck von im Saugkern gespeicherten, farbigen Körperflüssigkeiten abschwächt. Die Figuren 4 und 5 sollen dies verdeutlichen.

Figur 5 zeigt die dem Benutzer zugewandte Seite einer Slipeinlage, welche keinerlei Maßnahmen zur Verbesserung der Maskierungseigenschaften aufweist. Nach dem Aufsaugen farbiger Körperflüssigkeiten zeigt sich das Topsheet am Auftreffpunkt (6) dunkel gefärbt, die in der Saugschicht bereits absorbierten Anteile (7) färben den Saugkern um den Auftreffpunkt (6) herum konzentrisch an. Diese Verfärbung scheint durch das Topsheet. Der Benutzer einer derartigen Slipeinlage empfindet diesen Farbeindruck als unhygienisch.

Figur 4 zeigt den gleichen Ausschnitt, aber mit einem erfindungsgemäßen Vliesstoff (1) als Topsheet. Auch hier verfärbt sich Topsheet und Saugkern nach dem Auftreffen farbiger Körperflüssigkeiten. Die das Ausgangsmaterial bildenden Fasern oder Filamente (4) weisen Bereiche (2) auf, in welchen eine hydrophobe, die Opazität erhöhende Auflage (9) appliziert wurde. Diese Auflage (9) ändert nun die Hydrophilie der Fasern (4), sodass diese nun im Gegensatz zu den Bereichen ohne Auflage (3) hydrophob sind und dadurch von auftreffenden Körperflüssigkeiten nicht angeschmutzt werden. Diese Bereiche (2) kommen, nachdem die Körperflüssigkeiten vom Saugkern aufgenommen wurden, deutlich zum Vorschein und bilden einen Kontrast zu der Farbe der Körperflüssigkeiten. In der Folge schwächt sich die durch die Körperflüssigkeiten hervorgerufene Farbtiefe ab, was der Anwender angenehm und hygienisch empfindet.

Die Abschwächung des Farbtiefeneindrucks wurde durch Testpersonen mittels subjektiver Bewertung der Opazität ermittelt und bewertet (Tabelle 4) Es wurde dabei eine Bewertungsskala verwendet, die folgende Abstufungen hat:

| | |
|---|---|
| Note 1: | sehr gute Opazität - sehr gute Abschwächung des Farbtiefeneindrucks, nicht mehr sichtbar. |
| Note 2: | gute Opazität |
| Note 3: | befriedigende Opazität |
| Note 4: | ausreichende Opazität |
| Note 5: | mangelhafte Opazität |
| Note 6: | ungenügende Opazität - keine Abschwächung des Farbtiefeneindrucks - entspricht Figur 5 |

Die Durchlässigkeit gegenüber Fluiden teilt sich auf in die Durchlässigkeit gegenüber flüssigen und gasförmigen Fluiden.

Die Durchlässigkeit gegenüber Flüssigkeiten ist wichtig zur Aufnahme der Körperflüssigkeiten, also ein wichtiger Parameter der Funktion des Topsheets. Der Tabelle 2 können Durchlaufzeiten des erfindungsgemäßen Materials entnommen werden. Sie wurde gemäß WSP 70.3 (05) ermittelt. Durchlaufzeiten von größer 15 Sekunden werden dabei als negativ bewertet.

Die Durchlässigkeit gegenüber gasförmigen Fluiden ist eine Eigenschaft, die während des Gebrauchs den Tragekomfort beeinflusst. Geringe Durchlässigkeit gegenüber gasförmigen Fluiden erzeugt beim Anwender das Gefühl einer plastikähnlichen Oberfläche und wird als negativ bewertet. Diese Eigenschaft wurde gemäß WSP 70.1 (05) ermittelt, ein Vergleich von unbehandeltem Material zu erfindungsgemäßem Vliesstoff (1) kann Tabelle 1 entnommen werden.

Die zugrunde liegenden Techniken der Herstellung von Vliesstoffen aus thermoplastischen Fasern - kardierten Vliesstoffen - oder thermoplastischen Filamenten - Spinnvliesstoffen-, sowie deren Verfestigung mittels Druck und Hitze in einem Kalander können dabei dem Standardwerk für die Vliesstofftechnik, dem Buch "Vliesstoffe", herausgegeben von Albrecht, Fuchs und Kittelmann, erschienen im Verlag Wiley VCH, 2000 entnommen werden.

Erfindungsgemäß kommt als Ausgangsmaterial ein aus thermoplastischen Fasern oder Filamenten bestehender hydrophiler Vliesstoff, der thermisch mittels eines Kalanders verfestigt wird, zum Einsatz. Anschließend werden die Oberflächen der Fasern (4) und / oder Teilbereiche deren Oberflächen mit einer Auflage (9) versehen, die zum einen die Hydrophobie dieser Bereiche (2) und zum anderen auch deren Opazität erhöht, ohne dass sich die Durchlässigkeit des erfindungsgemäßen Vliesstoffs (1) gegenüber flüssigen und gegenüber gasförmigen Fluiden ändert.

Die das Ausgangsmaterial bildenden, hydrophilen thermoplastischen Fasern oder Filamente (4) mit handels- bzw. verfahrensüblichen Fasertitern zwischen 0,5 und 12dtex sind nur an wenigen Stellen bezogen auf die Gesamtfläche des Ausgangsmaterials miteinander thermisch mittels eines Kalanders verschmolzen. Der Anteil der Verfestigungsflächen, sogenannte Kalanderprints, ist verhältnismäßig gering gehalten. Somit hat der Vliesstoff nur wenige, stark verdichtete Bereiche, Als geeignet erwiesen sich Pressflächen im Bereich zwischen 8 und 30 %, wobei der Bereich zwischen 10 und 16% besonders bevorzugt ist.

Pressflächen unterhalb 8% führen zu mechanisch instabilen Ausgangsmaterialien, Pressflächen über 30 % führen zu einem steifen Warenausfall, der für diese Art von Produkten nicht gewünscht ist.

Das Flächengewicht, gemessen nach WSP 130.1 (05), für dieses hydrophile Ausgangsmaterial liegt im Bereich von 7 - 50g/m², bevorzugt zwischen 20 und 35g/m².

Höhere Flächengewichte zeigen eine schlechtere Durchlässigkeit gegenüber flüssigen und gasförmigen Fluiden, geringere Flächengewichte haben zu wenig Fasermasse, was sich in unzureichender Opazität gegenüber den gefärbten Körperflüssigkeiten auswirkt.

Dieses Ausgangsmaterial wird nun einem Applikationssystem zugeführt, bei welchem nur auf den Oberflächen der Fasern (4) innerhalb der Bereiche (2) erfindungsgemäß und im Gegensatz zum Stand der Technik, eine hydrophobe, opake Substanz, die Auflage (9), aufgebracht wird.

Eine Filmbildung zwischen den Fasern (4), wie im Stand der Technik gemäß EP 633762 oder DE 4321155 beschrieben, findet nicht statt. Die Auflage (9) wird nur auf der Oberfläche der Fasern (4) innerhalb der Bereiche (2) erzeugt. Der erfindungsgemäße Vliesstoff (1) bleibt damit weiterhin für flüssige und gasförmige Fluide unverändert durchdringbar.

Beispielsweise haben sich Applikationssysteme als geeignet erwiesen, welche mustermäßig verteilt, mittels konzentrierter Sprühnebel die Auflagen (9) in den Bereichen (2) bilden können,

Dabei wird eine hydrophobe, opake Substanz, gelöst und/oder dispergiert in Wasser oder Lösungsmittel in Form eines konzentrierten, feinen Nebels, beispielsweise durch eine Düse nach dem Drop-on-Demand Verfahren versprüht. Das gewählte Lösungsmittel verdampft im Anschluss an Besprühung, zurück an den Fasern (4) innerhalb der Bereiche (2) bleibt nur die hydrophobe opake Auflage (9). Durch die Kombination vieler Düsen, die rechnergesteuert abwechselnd sprühen bzw. nicht sprühen, lassen sich auf den Fasern (4) Auflagen (9) innerhalb klar definierter, sich mustermäßig wiederholender Bereiche (2) mit hydrophober, opaker Auflage (9) erzeugen.

Die Wahl der zum Einsatz kommenden Systeme aus hydrophober, opaker Substanz und Lösungsmittel ist letztendlich von der Höhe der gewünschten Hydrophobie der so behandelten Bereichen (2) abhängig.

Auf Wasser basierende Systeme kommen zum Einsatz, wenn nur eine geringe Erhöhung der Hydrophobie der Bereiche (2) gewünscht wird. Ist eine größere Hydrophobie gewünscht, kommen auf Lösemittel basierende Systeme zum Einsatz. Zur Erhöhung der opaken Eigenschaften kann der der hydrophoben Substanz neben einem Farbmittel an sich zusätzlich noch beispielsweise Titandioxid beigegeben.

Erfindungsgemäß durchdringt der konzentrierte Sprühnebel das Ausgangsmaterial vollkommen. Dies wird unterstützt durch die sehr offenporige Struktur, und die wenigen vorhandenen Kalanderprints des Ausgangsmaterials.

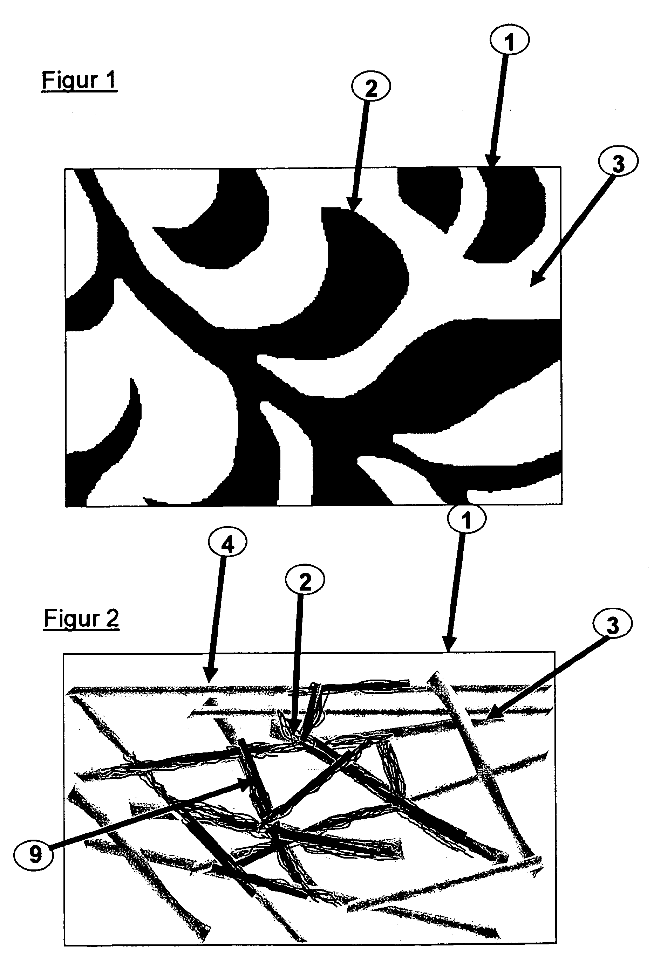

Erfindungsgemäß scheidet sich die hydrophobe, opake Substanz dabei nur auf der Oberfläche der Fasern (4) innerhalb der Bereiche (2) ab ohne dabei Poren, die zwischen den einzelnen Fasern liegen, zu versiegeln. Die bereits im Ausgangsmaterial vorhandenen Poren bleiben nahezu vollständig erhalten. Körperflüssigkeiten und Luft können diese Poren weiterhin ungehindert passieren, das Anschmutzen der mit einer Auflage (9) versehenen Fasern innerhalb der Bereiche (2) wird verhindert. Durch die Auflage (9) auf der Faseroberfläche wird die Opazität der Fasern (4) erhöht. Die Bereiche (2) haben daher im Vergleich zu den Bereichen (3) eine höhere Opazität Ein erfindungsgemäßes behandeltes Material (1) kann der Figur 2 entnommen werden

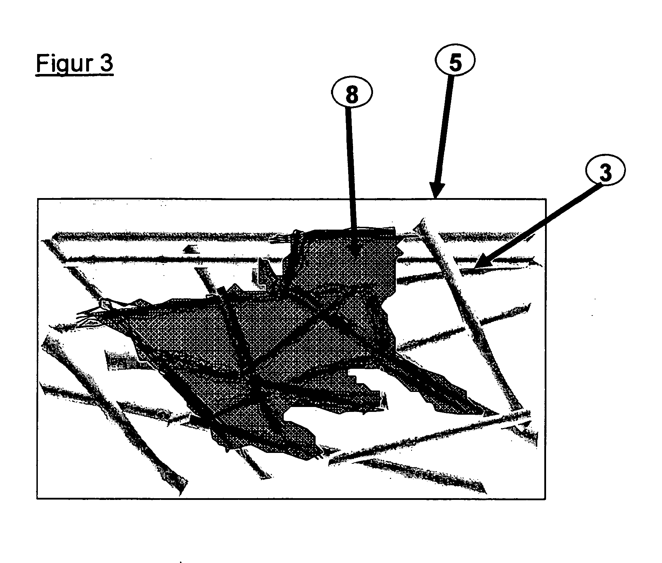

Im Unterschied dazu zeigt Figur 3 ein Material nach dem Stand der Technik. Dort werden die Poren zwischen den einzelnen Fasern verschlossen (8). Luft- und Flüssigkeitdurchlässigkeit gehen zurück, da durch die Versiegelung weniger offene Porenfläche zur Verfügung steht.

Die Durchlässigkeit eines erfindungsgemäßen Vliesstoffes (1) gegenüber gasförmigen Fluiden unterscheidet sich nicht von der des Ausgangsmaterials. Dies zeigt Tabelle 1. Hier wurde die Durchlässigkeit des Ausgangsmaterials gegenüber gasförmigen Fluiden, ausgedrückt als Luftdurchlässigkeit, mit der des erfindungsmäßen Vliesstoffes (1) verglichen. Die beiden Mittelwerte sind annähernd gleich.

Im Unterschied zum Stand der Technik können Fluide beim erfindungsgemäßen Vliesstoff (1) neben den Bereichen ohne Auflage (3) auch die Bereiche (2) mit Auflage weiterhin passieren. Das Anschmutzen der Fasern (4) durch Körperflüssigkeiten wird in den Bereichen mit Auflage (2) durch den hydrophoben Charakter der Auflage (9) vermieden.

In Abhängigkeit von der Anzahl Fasern, die so im Vlies behandelt wurden, und der Menge an Auflage (9) lassen sich unterschiedliche Hydrophobien und Opazitäten erzielen.

Um diese Abhängigkeiten zu verdeutlichen, wurde ein Basisvlies mit verschiedenen Anteilen behandelter Fasern, somit auch verschiedenen Mengen der Auflage (9) hergestellt. Ermittelt wurde die Menge an Auflage (9) über den Verbrauch von Lösemittel mit darin enthaltener hydrophober, opaker Substanz. Der Gehalt an hydrophober, opaker Substanz im Lösemittel war dabei bekannt.

Das folgende Ausführungsbeispiel bezieht sich auf den Anwendungsbereich Topsheet, wobei hier folgende Materialien zum Einsatz kamen:

| | |
|---|---|
| Ausgangsmaterial: | kardiertes Stapelfaservlies aus 100% PP-Fasern, 2.2dtex/40mm, hydrophil, Flächengewicht 27 g/m², thermisch kalanderverfestigt, Pressfläche 11 %, Dicke 0,62mm (WSP 120.6(05), 0,02kPa Vorlast) |
| Hydrophobe, opake Substanz: | lösemittelbasierendes System UN 1263 + Farbstoff Titandioxid, Gehalt an Trockensubstanz ca. 20% |

An den Mustern wurde dann die Durchlässigkeit gegenüber flüssigen und gasförmigen Fluiden bestimmt und die Opazität subjektiv bewertet.

Die Tabelle 2 zeigt das Verhalten der Durchlässigkeit von Flüssigkeiten des erfindungsgemäßen Vliesstoffs (1) bei steigendem Anteil von Bereichen (2) mit Auflage (9). Die Auflagemenge an hydrophober, opaker Substanz wurde dabei von Versuch zu Versuch um jeweils ca. 0,25 g/m² erhöht. Die Durchlässigkeit wurde gemäß WSP 70.3 (05) ermittelt und als Durchlasszeit in Sekunden angegeben.

Es zeigte sich, dass bei dem vorliegenden Beispiel bis zu einer Auflagemenge von ca. 1,75 g/m² die Durchlasszeiten unterhalb der als kritisch angesehenen Grenze von 15 Sekunden liegen.

Der Tabelle 3 kann entnommen werden, das dieses Verhalten bei der Durchlässigkeit gegenüber gasförmigen Fluiden (gemessen nach WSP 70.1 (05), angegeben in l/m²*s) ähnlich ist. Auch hier ist über einen weiten Bereich, bis zu einer Auflage von ca. 1,75g/m² keine Beeinträchtigung der Durchlässigkeit zu erkennen.

Zum Zwecke der Beurteilung der Opazität wurden Musteraufbauten bestehend aus dem erfindungsgemäßen Vliesstoff (1) als Topsheet und einem nachfolgenden absorbierenden Saugmaterial aus einem Airlaidmaterial mit ca. 80 g/m² Flächengewicht hergestellt. Auf diesen Aufbau wurden dann 5ml Schafsblut aufgetragen und 15 Minuten nach dem Eindringen die Opazität gemäß der eingangs erwähnten Skala durch 20 Probanden bewertet. Die subjektive Einstufung kann der Tabelle 4 entnommen werden.

Es zeigt sich, dass erst ab einer Auflagemenge von ca. 0,75 g/m² die Opazität als "ausreichend" bewertet wird. Je mehr Menge der Auflage (9) vorhanden ist, umso besser wird die Opazität und damit die Eigenschaft zur Abschwächung der Farbtiefe angesehen.

Größere Mengen der Auflage (9) von bis zu 10 g/m² lassen sich mit höhergewichtigen Ausgangsvliesstoffen und feineren Fasern wie der hier eingesetzten 2.2dtex PP-Fasern realisieren. Letztendlich bestimmt der Anwendungszweck die Kombination aus Flächengewicht des Ausgangsmaterials, Feinheit der Fasern und Menge der Auflage (9) zur Erreichung der optimalen Balance aus Flüssigkeitstransport, Maskierungseigenschaften, Atmungsaktivität und Weichheit/ Tragekomfort.

Überraschend wurde festgestellt, dass die Probanden eine Auflage (9) als wenig attraktiv und unhygienisch betrachteten, die eine andere Farbe als das Ausgangsmaterial bzw. die das Ausgangsmaterial bildenden Fasern (4) hatten. Die Mehrheit der Probanden empfand eine zum Ausgangsmaterial farbgleiche Auflage (9) beispielsweise weiße Fasern mit weißer Auflage (9), die erst im Gebrauch durch Kontrastbildung in Erscheinung tritt, als angenehm und hygienisch. Auch zeigte sich, das geometrische Muster wie Gitterlinien oder Punktmuster weniger Akzeptanz im Vergleich zu bildlichen Mustern, wie floralen Motiven, beispielsweise in Figur 1 dargestellt, oder Spitzenmustern hatten.

**Tabelle 1: Vergleich der Luftdurchlässigkeit (gemäß WSP 70.1 (05))**

| | LD bei Differenzdruck von 100 Pa und 20qcm Messfläche [l/(m²*s)] |
|---|---|
| **Ausgangsmaterial** Vlies aus 100% hydrophiler PP-Faser 2.2.dtex / 40mm, Verfestigungsfläche 11 %, Gewicht 27 g/ m,² | 2550 |
| | 2420 |
| | 2420 |
| | 2360 |
| | 3160 |
| Mittelwert | 2582 |
| **Erfindungsgemäßes Vlies (1)** Entsprechend Ausgangsmaterial + Auflage (9) ca 1 g/ m² | 2440 |
| | 2970 |
| | 2720 |
| | 2480 |
| | 2410 |
| Mittelwert | 2604 |

**Tabelle 2: Durchlässigkeiten gegenüber flüssigen Fluiden (gemäß WSP 70.3 (05))**

| | | | | | | | | | |
|---|---|---|---|---|---|---|---|---|---|
| | **hydrophobe, opake Auflage (9) in g/ m²** | | | | | | | | |
| | **0,25** | **0,50** | **0,75** | **1,00** | **1,25** | **1,50** | **1,75** | **2,00** | **2,50** |
| 1 | 2,1 | 2,4 | 3,2 | 3,6 | 4,9 | 10,3 | 12,2 | 15,6 | 18,3 |
| 2 | 2,0 | 2 | 2,4 | 2,6 | 5 | 8,7 | 12,8 | 17,3 | 22,8 |
| 3 | 2,0 | 2,6 | 2,7 | 3,4 | 6,1 | 11,8 | 13,1 | 15,4 | 20,8 |
| 4 | 2,3 | 2,4 | 2,6 | 2,5 | 4,6 | 9,7 | 11,1 | 16,3 | 21,5 |
| 5 | 2,2 | 3,1 | 2,4 | 2,7 | 5,1 | 8,5 | 14,5 | 18,7 | 19,6 |
| **Mittelwert In Sekunden** | **2,1** | **2,5** | **2,7** | **3,0** | **5,1** | **9,8** | **12,7** | **16,7** | **20,6** |

**Tabelle 3: Durchlässigkeiten gegenüber gasförmigen Fluiden (gemäß WSP 70.1 (05))**

| | **hydrophobe, opake Auflage (9) in g/ m²** | | | | | | | | |
|---|---|---|---|---|---|---|---|---|---|
| | **0,25** | **0,50** | **0,75** | **1,00** | **1,25** | **1,50** | **1,75** | **2,00** | **2,50** |
| 1 | 2660 | 2730 | 2490 | 2440 | 2550 | 2360 | 2420 | 2380 | 1980 |
| 2 | 2750 | 2800 | 2350 | 2970 | 2590 | 2510 | 2370 | 2160 | 1910 |
| 3 | 2840 | 2390 | 2750 | 2720 | 2390 | 2550 | 2190 | 2100 | 2210 |
| 4 | 2520 | 2470 | 2550 | 2480 | 2470 | 2490 | 2390 | 2240 | 2060 |
| 5 | 2430 | 2750 | 2640 | 2410 | 2730 | 2850 | 2470 | 2000 | 2030 |
| **Mittelwert in** l/m² *s | **2640** | **2628** | **2556** | **2604** | **2546** | **2552** | **2368** | **2176** | **2038** |

**Tabelle 4: Visuelle Bewertung der Maskierungseigenschaft**

| **Proband** | **hydrophobe, opake Auflage (9) in g/ m²** | | | | | | | | |
|---|---|---|---|---|---|---|---|---|---|
| | **0,25** | **0,50** | **0,75** | **1,00** | **1,25** | **1,50** | **1,75** | **2,00** | **2,50** |
| 1 | 6 | 6 | 5 | 3 | 3 | 2 | 2 | 1 | 1 |
| 2 | 6 | 5 | 4 | 4 | 4 | 2 | 2 | 2 | 1 |
| 3 | 6 | 5 | 5 | 1 | 2 | 2 | 2 | 1 | 1 |
| 4 | 6 | 6 | 5 | 4 | 4 | 2 | 2 | 2 | 1 |
| 5 | 6 | 5 | 4 | 6 | 3 | 2 | 1 | 2 | 1 |
| 6 | 6 | 6 | 4 | 5 | 3 | 3 | 2 | 1 | 1 |
| 7 | 6 | 5 | 4 | 4 | 4 | 2 | 2 | 2 | 1 |
| 8 | 6 | 5 | 5 | 4 | 3 | 2 | 2 | 2 | 1 |
| 9 | 6 | 5 | 4 | 3 | 3 | 2 | 2 | 2 | 1 |
| 10 | 6 | 6 | 5 | 3 | 3 | 3 | 2 | 1 | 1 |
| 11 | 6 | 5 | 5 | 5 | 2 | 2 | 2 | 2 | 1 |
| 12 | 6 | 5 | 4 | 4 | 3 | 2 | 3 | 2 | 1 |
| 13 | 6 | 5 | 4 | 5 | 3 | 2 | 3 | 2 | 1 |
| 14 | 6 | 6 | 4 | 1 | 3 | 2 | 2 | 2 | 1 |
| 15 | 6 | 6 | 4 | 5 | 3 | 2 | 2 | 3 | 1 |
| 16 | 6 | 5 | 5 | 4 | 2 | 2 | 1 | 2 | 1 |
| 17 | 6 | 6 | 5 | 4 | 3 | 2 | 2 | 1 | 1 |
| 18 | 6 | 5 | 4 | 5 | 4 | 3 | 2 | 2 | 1 |
| 19 | 6 | 5 | 5 | 4 | 3 | 3 | 2 | 2 | 1 |
| 20 | 6 | 6 | 5 | 5 | 3 | 2 | 2 | 2 | 1 |
| **Mittelwert** | **6,0** | **5,4** | **4,5** | **4,0** | **3,1** | **2,2** | **2,0** | **1,8** | **1,0** |

### Bezugszeichen-Liste

1 = erfindungsgemäßes Material
2 = Bereich von Fasern mit hydrophober, opaker Auflage
3 = Bereich von Fasern ohne hydrophobe, opake Auflage
4 = Fasern
5 = Material gemäß Stand der Technik
6 = Auftreffpunkt
7 = vom Saugkern absorbierte, farbige Körperflüssigkeit
8 = versiegelter Bereich
9 = hydrophobe, opake Auflage

## Patentansprüche

1. **Topsheet** für einen **Hygieneartikel** aus hydrophilen Fasern oder Filamenten mit verbesserten Maskierungseigenschaften gegenüber Körperflüssigkeiten, **bestehend aus einem Vliesstoff,** wobei der Vliesstoff mustermäßig aufgebrachte, hydrophobe Bereiche (2) hat,
**dadurch gekennzeichnet,**
**dass der Vliesstoff (1) innerhalb der Bereiche (2) Fasern (4) aufweist, die wenigstens partiell eine hydrophobe Auflage (9) haben, und,**
**dass die Bereiche (2) luft- und flüssigkeitsdurchlässig sind.**

2. **Topsheet** nach Anspruch 1,
**dadurch gekennzeichnet,**
**dass** die hydrophobe Auflage (9) opak ist.

3. **Topsheet** nach einem der Ansprüche 1 und 2,
**dadurch gekennzeichnet,**
**dass** der Vliesstoff (1) im trockenen Zustand mit einer Menge der Auflage (9) von maximal 10 g/**m²** beaufschlagt ist.

4. **Topsheet** nach einem der Ansprüche 1 bis 3,
**dadurch gekennzeichnet,**
**dass** das Flächengewicht des Vliesstoffes (1) mindestens 7 bis maximal 50 g/**m²** und bevorzugt mindestens 20 bis maximal 35 g/**m²** hat.

5. **Topsheet** nach einem der Ansprüche 1 bis 4,
**dadurch gekennzeichnet,**
**dass** die den Vliesstoff (1) bildenden Fasern oder Filamente (4) eine Feinheit von mindestens 0,5 dtex aufweisen.

6. **Topsheet** nach einem der Ansprüche 1 bis 5,
**dadurch gekennzeichnet,**
**dass** die den Vliesstoff (1) bildenden Fasern oder Filamente (4) aus thermoplastischen Polymeren bestehen

7. **Topsheet** nach einem der Ansprüche 1 bis 6,
**dadurch gekennzeichnet,**
**dass** die Auflage (9) auf Lösemittel basierend ist.

8. **Topsheet** nach einem der Ansprüche 1 bis 7,
**dadurch gekennzeichnet,**
**dass** die Auflage (9) auf Wasser basierend ist.

9. **Topsheet** nach einem der Ansprüche 1 bis 8,
**dadurch gekennzeichnet,**
**dass** die Auflage (9) ein Mattierungsmittel enthält.

10. **Topsheet** nach einem der Ansprüche 1 bis 9,
**dadurch gekennzeichnet,**
**dass** die Auflage (9) gleichfarbig mit den Fasern oder Filamenten (4) ist.

11. **Topsheet** nach einem der Ansprüche 1 bis 10,
**dadurch gekennzeichnet,**
**dass** der Vliesstoff (1) eine Luftdurchlässigkeit von mindestens 500 l/(m² *sec) aufweist.

12. **Topsheet** nach einem der Ansprüche 1 bis 11,
**dadurch gekennzeichnet,**
**dass** der Vliesstoff (1) aus kardierten und mittels beheizter Kalanderprägewalzen thermobondierten Fasern besteht und,
**dass** der Vliesstoff eine thermobondierte Prägefläche von 8% bis 30% aufweist.

13. **Topsheet** nach einem der Ansprüche 1 bis11,
**dadurch gekennzeichnet,**
**dass** der Vliesstoff (1) aus mittels beheizter Kalanderprägewalzen thermobondierten Filamenten besteht und,
**dass** der Vliesstoff (1) eine thermobondierte Prägefläche von 8% bis 30% aufweist.

14. Hygieneartikel, insbesondere Damenbinde, enthaltend **ein Topsheet** aus hydrophilen Fasern oder Filamenten mit verbesserten Maskierungseigenschaften gegenüber Körperflüssigkeiten, wobei das **Topsheet aus Vliesstoff besteht und** mustermäßig aufgebrachte, hydrophobe **Bereiche (4)** hat,
**dadurch gekennzeichnet,**
**dass der Vliesstoff (1) innerhalb der Bereiche (4) Fasern aufweist, die wenigstens partiell eine hydrophobe Auflage (9) haben, und, dass die Bereiche (4) luft- und flüssigkeitsdurchlässig sind.**

15. Medizinischer Artikel, insbesondere Wundauflage, enthaltend **ein Topsheet** für Hygienezwecke aus hydrophilen Fasern oder Filamenten mit verbesserten Maskierungseigenschaften gegenüber Körperflüssigkeiten, wobei **das Topsheet aus Vliesstoff besteht und** mustermäßig aufgebrachte, hydrophobe **Bereiche (4)** hat,
**dadurch gekennzeichnet,**
**dass der Vliesstoff (1) innerhalb der Bereiche (4) Fasern aufweist, die wenigstens partiell eine hydrophobe Auflage (9) haben, und, dass die Bereiche (4) luft- und flüssigkeitsdurchlässig sind.**

## Claims

1. A top sheet for a hygiene article made of hydrophilic fibres or filaments with improved masking properties with respect to body fluids, consisting of a fleece material, whereby the fleece material has hydrophobic areas (2) applied in patterns,
**characterised in that**
within the areas (2) the fleece material (1) features fibres (4), which at least partially have a hydrophobic layer (9), and that the areas (2) are permeable to air and to liquid.

2. The top sheet according to claim 1
**characterised in that**
the hydrophobic layer (9) is opaque.

3. The top sheet according to one of claims 1 and 2,
**characterised in that**
in the dry state, the fleece material (1) is provided with maximum 10 g/m2 of the layer (9).

4. The top sheet according to one of claims 1 to 3,
**characterised in that**
the weight per unit area of the fleece material (1) has at least 7 to maximum 50 g/m2 and preferably at least 20 to maximum 35 g/m2.

5. The top sheet according to one of claims 1 to 4,
**characterised in that**
the fibres or filaments (4) forming the fleece material (1) feature fineness of at least 0.5 dtex.

6. The top sheet according to one of claims 1 to 5,
**characterised in that**
the fibres or filaments (4) forming the fleece material (1) consist of thermoplastic polymers.

7. The top sheet according to one of claims 1 to 6,
**characterised in that**
the layer (9) is solvent based.

8. The top sheet according to one of claims 1 to 7,
**characterised in that**
the layer (9) is water based.

9. The top sheet according to one of claims 1 to 8,
**characterised in that**
the layer (9) contains a delustrant.

10. The top sheet according to one of claims 1 to 9,
**characterised in that**
the layer (9) is of the same colour as the fibres or filaments (4).

11. The top sheet according to one of claims 1 to 10,
**characterised in that**
the fleece material (1) features air permeability of at least 500 l/(m2*sec).

12. The top sheet according to one of claims 1 to 11,
**characterised in that**
the fleece material (1) consists of carded fibres and is thermally bonded by means of heated calender roll embossing and, the fleece material features a thermally bonded embossed surface area of 8% to 30%.

13. The top sheet according to one of claims 1 to 11,
**characterised in that**
the fleece material (1) consists of thermally bonded filaments, by means of heated calender roll embossing and,
the fleece material features a thermally bonded and embossed surface area of 8% to 30%.

14. A hygienic article, particularly sanitary towel, containing a top sheet made of hydrophilic fibres or filaments with improved masking properties with respect to body fluids, whereby the top sheet consists of fleece material and has hydrophobic areas (4) applied in patterns,
**characterised in that**
within the areas (4), the fleece material (1) features fibres which at least partially have a hydrophobic layer (9), and that the areas (4) are permeable to air and to liquid.

15. A medical article, particularly wound dressing, containing a top sheet for hygienic purposes made of hydrophilic fibres or filaments with improved masking properties with respect to body fluids, whereby the top sheet consists of fleece material and has hydrophobic areas (4) applied in patterns,
**characterised in that**
within the areas (4), the fleece material (1) features fibres which at least partially have a hydrophobic layer (9), and that the areas (4) are permeable to air and to liquid.

## Revendications

1. Feuille supérieure pour un article d'hygiène en fibres ou filaments hydrophiles offrant des propriétés améliorées de masquage des liquides corporels, composée d'un non-tissé, sachant que le non-tissé présente des zones (2) hydrophobes disposées selon un motif,
**caractérisée en ce que**
le non-tissé (1) présente, à l'intérieur des zones (2), des fibres (4) comportant au moins partiellement une garniture (9) hydrophobe,
et **en ce que** ces zones (2) sont perméables à l'air et au liquide.

2. Feuille supérieure selon la revendication 1,
**caractérisée en ce que**
la garniture (9) hydrophobe est opaque.

3. Feuille supérieure selon l'une des revendications 1 et 2,
**caractérisée en ce que**
le non-tissé (1) à l'état sec se trouve recouvert d'une quantité de la garniture (9) s'élevant au maximum à 10 g/m².

4. Feuille supérieure selon l'une des revendications 1 à 3,
**caractérisée en ce que**
le grammage du non-tissé (1) est compris entre au minimum 7 et au maximum 50 g/m², et préférentiellement entre au minimum 20 et au maximum 35 g/m².

5. Feuille supérieure selon l'une des revendications 1 à 4,
**caractérisée en ce que**
les fibres ou filaments (4) constitutifs du non-tissé (1) présentent une finesse d'au moins 0,5 dtex.

6. Feuille supérieure selon l'une des revendications 1 à 5,
**caractérisée en ce que**
les fibres ou filaments (4) formant le non-tissé (1) se composent de polymères thermoplastiques

7. Feuille supérieure selon l'une des revendications 1 à 6,
**caractérisée en ce que**
la garniture (9) est à base de solvants.

8. Feuille supérieure selon l'une des revendications 1 à 7,
**caractérisée en ce que**
la garniture (9) est à base d'eau.

9. Feuille supérieure selon l'une des revendications 1 à 8,
**caractérisée en ce que**
la garniture (9) contient un produit matifiant.

10. Feuille supérieure selon l'une des revendications 1 à 9,
**caractérisée en ce que**
la garniture (9) est de la même teinte que les fibres ou filaments (4).

11. Feuille supérieure selon l'une des revendications 1 à 10,
**caractérisée en ce que**
le non-tissé (1) présente une perméabilité à l'air d'au moins 500 l/(m² *sec)

12. Feuille supérieure selon l'une des revendications 1 à 11,
**caractérisée en ce que**
le non-tissé (1) se compose de fibres cardées et thermoliées au moyen de cylindres de gaufrage et calandrage chauffés, et **en ce que**
le non-tissé présente une surface gaufrée thermoliée comprise entre 8 et 30%.

13. Feuille supérieure selon l'une des revendications 1 à 11,
**caractérisée en ce que**
le non-tissé (1) se compose de filaments thermoliés au moyen de cylindres de gaufrage et calandrage chauffés, et **en ce que**
le non-tissé (1) présente une surface gaufrée thermoliée comprise entre 8 et 30%.

14. Article d'hygiène, en particulier serviette périodique, contenant une feuille supérieure en fibres ou filaments hydrophiles offrant des propriétés améliorées de masquage des liquides corporels, sachant que la feuille supérieure est en non-tissé et qu'elle présente des zones (4) hydrophobes disposées selon un motif,
**caractérisé en ce que**
le non-tissé (1) présente, à l'intérieur des zones (4), des fibres comportant au moins partiellement une garniture (9) hydrophobe, et que ces zones (4) sont perméables à l'air et au liquide.

15. Article médical, en particulier pansement, contenant une feuille supérieure à des fins d'hygiène en fibres ou filaments hydrophiles offrant des propriétés améliorées de masquage des liquides corporels, sachant que la feuille supérieure est en non-tissé et qu'elle présente des zones (4) hydrophobes disposées selon un motif,
**caractérisé en ce que**
le non-tissé (1) présente, à l'intérieur des zones (4), des fibres comportant au moins partiellement une garniture (9) hydrophobe, et **en ce que** ces zones (4) sont perméables à l'air et au liquide.
